# NOUVEAU FASCICULE DE BREVET EUROPEEN

(11) **EP 0 237 427 B2**
(45) Date de publication et mention de la décision concernant l'opposition: **19.03.1997**
(45) Mention de la délivrance du brevet: 19.09.1990
(21) Numéro de dépôt: 87400510.1
(22) Date de dépôt: 09.03.1987
(51) Int. Cl.: C12N 1/04, A21D 6/00

(54) **Levures surgelées en particules fluides s'écoulant librement, procédé pour les obtenir et application de ces levures aux pâtes congelées**
Gefrorene Hefe in Form von frei fliessenden Teilchen, Verfahren zu deren Herstellung und Verwendung dieser Hefen in gefrorenen Teigen
Frozen yeasts in fluid free-flowing particles, process for obtaining them and use of these yeasts in frozen doughs

(30) Priorité: 07.03.1986 US 840510
(43) Date de publication de la demande: 16.09.1987
(73) Titulaire: LESAFFRE et Cie, F-75001 Paris (FR)
(72) Inventeur: Goux, Jean, F-59700 Marcq en Baroeul (FR); Clément, Philippe, F-59100 Roubaix (FR)
(74) Mandataire: Koch, Gustave

(56) Documents cités:
- EP-A- 0 153 117
- DD-A- 249 153
- FR-A- 2 148 727
- GB-A- 634 919
- GB-A- 900 808
- US-A- 3 089 774
- C Khlebopekarnaya i Konditerskaya Promyshlennost. vol 13 pp 35-36, 1969.
- Cryo-Lett 5(3) pp. 191-200. 1984
- Food Technology 3, pp. 234-6. 1949
- Microbial Technology 1967 pp. 156-157.
- The Yeasts vol. 3 pp. 395-398. 1970.
- Die Hefen, Band II.pp. 582-585. 1962

## Description

L'invention a pour objet une nouvelle présentation des levures, combinant les avantages des levures fraîches et des levures sèches. Elle vise une levure surgelée ou congelée ayant des matières sèches entre 70 et 85%, et encore de préférence entre 72 et 80% de matières sèches se présentant sous forme de particules, s'écoulant librement et incorporables directement dans les pâtes.

Les susdites levures conservent pratiquement indéfiniment et au moins pendant trois mois les propriétés de la levure fraîche de départ et sont susceptibles d'être obtenues par:
- division d'une levure fraîche, riche en tréhalose et ayant ses membranes intactes, par extrusion en particules individuelles de moins de 3 mm et, de préférence, de moins de 1 mm,
- séchage des particules ainsi obtenues jusqu'à une teneur en matières sèches comprise entre 70 et 85% par un séchage ménageant,
- refroidissement rapide des particules séchées jusqu'à une température comprise entre -1° et +4°C,
- surgélation ou congélation des particules ainsi refroidies par fluidisation dans un courant d'air à température négative comprise entre -10° et -40°C.

De préférence, elles donnent, dans les tests A₁, un dégagement gazeux compris entre 159 et 170 ml en deux heures.

L'invention a également pour objet le procédé d'obtention desdites nouvelles levures et leur application à la fabrication des pâtes congelées.

Le susdit procédé est caractérisé par le fait:
- qu'on divise une levure fraîche riche en tréhalose et ayant ses membranes intactes, par extrusion en particules individuelles de moins de 3 mm, de préférence de moins de 1 mm,
- qu'on sèche les particules ainsi obtenues jusqu'à une teneur en matières sèches comprise entre 70 et 85% par un séchage ménageant,
- qu'on refroidit rapidement les particules séchées à une température comprise entre -1° et +4°C,
- qu'on surgèle ou congèle les particules refroidies par fluidisation dans un courant d'air à une température négative comprise entre -10° et -40°C.

Les levures de panification sont commercialisées essentiellement sous deux formes:
■ sous forme de levure fraîche ayant entre 27 et 35% de matières sèches présentées en blocs de 500 g ou 1 kg, ou sous forme divisée (bulk yeast or crumbled yeast) en sacs de 11 à 15 kg. Pour que la levure fraîche reste sous forme divisée et ne se réagglomère pas, il est souvent nécessaire de lui ajouter un certain nombre d'additifs comme indiqué dans les brevets britanniques 1.530.866 et 1.560.478 ou la demande de brevet européen N° 0.153.117. Cette levure fraîche conservée à 4°C doit être utilisée au plus tard sous un mois et, de préférence, dans un délai de deux semaines. En pratique, les conditions de stockage des levures fraîches sont rarement idéales, et cette température de référence de 4°C est mal respectée. A côté de la levure fraîche devant être conservée à +4°C, il existe de manière marginale une commercialisation de levure fraîche en bloc de 500 g ou 1 kg sous forme congelée. La congélation conduit obligatoirement à des blocs de levures, la levure divisée en vrac, même avec additifs, s'agglomère en blocs lors de la congélation. Cette levure doit être décongelée pour être incorporée dans les pâtes et utilisée très rapidement après décongélation. Le traitement de congélation-décongélation est légèrement pénalisant pour la levure et l'emploi des levures congelées en bloc est mal commode,
■ sous forme de levure sèche à au moins 92% de matières sèches. Les levures sèches ont l'inconvénient d'avoir perdu une partie de leur activité au cours du séchage et les membranes des cellules de levures sont plus ou moins altérées. Elles ont l'avantage d'une longue conservation, les levures sèches instantanées les plus performantes, conditionnées sous vide ou gaz neutre, ont une perte d'activité fermentative de l'ordre de 1% par mois à 20°C. Ces levures sèches doivent toujours être réhydratées dans une pâte ou dans un liquide à au moins 16°C.

Dans le passé, on peut noter (brevet US 3 089 774) un essai de mise au point d'une levure à humidité intermédiaire, mais cette levure à humidité intermédiaire n'est pas produite au moins dans les pays de l'Ouest (Yeast Technology 1973, Reed et Peppler, page 94), ses qualités de conservation ne sont pas suffisamment supérieures à celles des levures fraîches à environ 30% de matières sèches, conservées à +4°C. De plus, ces levures à humidité intermédiaire sont très sensibles à une élévation de température.

Le brevet britannique GB-A-634 919 décrit un procédé d'atomisation de crème de levure, les gouttelettes ainsi obtenues étant envoyées dans de l'air froid. Ce procédé présente l'inconvénient résidant dans le taux de survie très faible des cellules de levure ainsi traitées.

L'invention consiste à surgeler ou à congeler par fluidisation une levure divisée, de manière à porter les particules de levure d'une température nulle ou positive à une température entre -5°C et -30°C, de préférence à -18°C ou -20°C en un temps compris entre 5 minutes et plusieurs heures, par exemple entre 7 et 120 minutes ou entre 10 et 40 minutes.

La surgélation ou la congélation par fluidisation dans un courant d'air ou de gaz neutre entre -10°C et -40°C, de préférence entre -20°C et -40°C, et encore de préférence entre -18°C et -40°C ainsi qu'entre -18°C et -30°C, permet d'une part des transferts de chaleur rapides entre le gaz et le solide (les particules de levure en lit fluidisé) sans faire appel à des températures très basses destructrices pour la levure et, d'autre part, l'individualisation des particules qui ne viennent pas se réagglomérer. C'est cette dernière propriété de la fluidisation qui est la plus intéressante, étant donné que la levure supporte aisément une descente lente en température et qu'il est préférable que la surgélation ne soit pas trop rapide.

Ce procédé peut à la rigueur s'appliquer à des levures pressées entre 30 et 40% de matières sèches, de préférence à au moins 35% de matières sèches, produites de manière à être non collantes et donc à pouvoir se fluidiser sans prendre en masse. Un grand soin doit être apporté dans la conception du congélateur en lit fluidisé et dans les opérations de mise en fluidisation de ces levures pressées de manière à obtenir des particules bien individualisées. La surgélation ne doit pas être trop rapide, la descente en température en dessous de 0°C ne doit pas être de plus d'un degré par minute. Les produits obtenus auront toujours tendance à motter, voire à prendre en masse, ce qui est un inconvénient important.

Ce procédé s'applique préférentiellement à des levures préséchées qui ne présentent aucune difficulté de mise en fluidisation. Le préséchage aura également pour avantage de diminuer les frais de stockage et de transport de ces levures surgelées du fait de leur plus faible poids. Un préséchage à au moins 72% de matières sèches et, de préférence, à au moins 74% de matières sèches garantira contre tout risque de réagglomération dans les emballages et fera que les particules de levures resteront parfaitement fluides.

Ce préséchage pourra être conduit jusqu'à l'apparition de dégradations des membranes cellulaires du fait de l'élimination de l'eau interne des cellules de levures. Il ne devra jamais dépasser 85% de matières sèches et, de préférence, il sera conduit jusqu'à environ 77% de matières sèches, c'est-à-dire afin d'obtenir des levures entre 74% et 80% de matières sèches.

La levure préséchée entre 70 et 85% de matières sèches devra être aussi rapidement que possible refroidie à une température comprise entre -1°C et +4°C, car les levures à ces matières sèches ont tendance à s'échauffer rapidement et elles ne doivent pas être laissées à des températures supérieures à 15°C. Ensuite, la vitesse de surgélation ou de congélation de la levure n'est pas un paramètre critique si elle n'est pas excessivement rapide. En d'autres mots, dès que ladite vitesse dépasse 5 à 10 minutes, on ne note pas de différence de propriétés de la levure, si on passe de 0°C à -20°C en 10 minutes ou 12 heures.

La surgélation ou la congélation en lit fluidisé de levure préséchée entre 70 et 85% de matières sèches, de préférence entre 72 et 80% de matières sèches, ne contenant donc plus d'eau externe ou d'eau faiblement liée, diminue considérablement le risque d'une cristallisation pouvant porter atteinte à l'intégralité des cellules.

Les levures surgelées ou congelées dans leur forme préférée entre 70% et 85% de matières sèches, de préférence entre 72% et 80% de matières sèches, et encore de préférence entre 74% et 80% de matières sèches, ont les avantages:
. d'être aisément manipulables et incorporables directement dans la pâte sans décongélation préalable,
. de conserver pratiquement indéfiniment les propriétés des levures fraîches de départ, prises à la sortie de leur fabrication.

Les levures surgelées divisées en particules, objet de l'invention, sont particulièrement intéressantes pour la fabrication des pâtes congelées destinées à être conservées plusieurs mois, puis à être décongelées, fermentées et cuites. Les études faites dans nos laboratoires ont montré que la fabrication de ces pâtes congelées exige, pour obtenir des pains ayant de manière uniforme et reproductible, le développement souhaité, l'utilisation de levures riches en substances de réserve, notamment en tréhalose, et ayant des membranes intactes. Dans la pratique, compte tenu des circuits de distribution, les levures fraîches ne peuvent être utilisées que plusieurs jours après leur sortie usine, et souvent elles n'ont pas été parfaitement maintenues à 4°C, mais plutôt à des températures de l'ordre de 10°C. En conséquence, les résultats obtenus après décongélation des pâtes sont hétérogènes et peu satisfaisants. L'emploi d'une levure divisée en particules, surgelée par fluidisation dès sa fabrication permet de mettre en oeuvre une levure ayant gardé toutes ces propriétés, sans aucune altération et donc de remédier à ces inconvénients. L'emploi de ladite levure surgelée, divisée en particules, dans la pâte congelée, permet d'obtenir des résultats meilleurs et plus réguliers en panification.

Les levures surgelées finement divisées et fluides ont l'avantage de pouvoir être incorporées ou dispersées directement dans les pâtes froides destinées à la congélation, ce qui fait que les levures seront d'autant moins actives dans cette pâte avant sa congélation, une activité nulle ou très faible des levures avant congélation étant très souhaitable. Dans ce but, il sera intéressant d'ajouter la levure surgelée en particules fluides à 77% en fin de pétrissage, juste assez tôt pour qu'elle soit dispersée totalement dans la pâte. Cet ajout tardif de la levure ne pénalise pas le développement de la pâte, contrairement à l'ajout tardif de levure pressée à 30% de matières sèches, en effet l'eau contenue dans la levure pressée et qui est nécessaire au développement de la pâte, peut être ajoutée directement dès le début du pétrissage, puisque ces levures congelées particulaires sont partiellement déshydratées.

Il est à remarquer que les levures surgelées, finement divisées, sous leur forme préférée entre 70% et 85% de matières sèches, de préférence entre 72% et 80% de matières sèches, ne présentent pas significativement plus d'excrétion cellulaire en réhydratation à basse température que la levure fraîche à environ 32% de matières sèches de départ prise juste après sa fabrication et dispersée dans le même milieu, ce qui montre que leurs membranes sont intactes et fait leur intérêt dans la fabrication des pâtes congelées.

Les levures surgelées en lit fluidisé selon l'invention se présentent en particules de dimensions inférieures à 3 mm. De préférence, les levures surgelées selon l'invention à matières sèches comprises entre 70% et 85% de matières sèches se présentent en vermicelles d'un diamètre inférieur à 1 mm.

Les levures surgelées selon l'invention peuvent éventuellement contenir les additifs décrits dans les brevets britanniques 1.530.866 et 1.560.478 ou dans le brevet U.S. 4.232.045 ou dans la demande de brevet européen 153.117, comme par exemple la silice et ses dérivés. Elles peuvent éventuellement contenir également les additifs utilisés généralement pour le séchage, comme ceux décrits dans les brevets U.S. No 4.328.250, 4.370.420 et 4.396.632, comme par exemple le monostéarate de sorbitan, les esters de polyglycérol ou les esters citriques de mono- et diglycérides et/ou alginates, gommes, dérivés de cellulose. Elles peuvent également contenir du lactose, du lactosérum ou du lait écrémé, du sorbitol, du glycérol, de la gélatine, c'est-à-dire des substances connues comme ayant des propriétés cryoprotectrices. L'ajout de ces additifs représente des variantes de réalisation de l'invention mais n'est nullement indispensable.

Les levures surgelées, divisées en particules de dimensions, inférieures à 3 mm, selon l'invention, sont emballées sous air ou sous gaz neutre, l'emballage sous gaz neutre n'ayant d'intérêt que si la levure doit être conservée plus de 3 mois avant d'être utilisée. Les levures surgelées et divisées sont emballées dans des sacs de 1 à 25 kg, réalisés dans les matériaux utilisés communément pour l'emballage, par exemple des légumes surgelés, comme un polyéthylène avec acétate de vinyle, ce matériau comportant une feuille d'aluminium si la levure surgelée est emballée sous gaz neutre.

### EXEMPLE 1

On réalise une levure pressée comme indiqué à partir de la ligne 14 de la page 5 du brevet européen publié sous le No 8.554, ou comme indiqué dans l'exemple 2 du brevet U.S. No 4.370.420 ou dans les exemples du brevet U.S. No 4.396.632.

On obtient une levure à 33% de matières sèches et contenant 7,3% d'azote sur matières sèches. On lui ajoute une fine émulsion constituée d'esters de sorbitol ou d'esters de polyglycérol dans une proportion d'environ 1,5% des matières sèches levure. On l'extrude à travers une grille ayant des perforations de largeur 0,5 à 3 mm, de préférence 0,5 à 1 mm et on la sèche à environ 75-78% de matières sèches par un séchage court particulièrement ménageant, c'est-à-dire un séchage où la levure ne dépasse pas 35°C et, de préférence, ne dépasse pas 30°C.

Les vermicelles de levure à environ 75 à 78% de matières sèches sont transférés dans un fluidiseur vibré alimenté en air à -25°C par un groupe frigorifique, l'ensemble étant soigneusement calorifugé ou placé dans une enceinte thermostatée.

De manière générale, on peut employer un fluidiseur de conception identique à ceux utilisés habituellement pour le séchage des levures par fluidisation, la différence essentielle étant son alimentation avec un air à température négative. Le principe utilisé est le même: on fait traverser le produit solide: la levure en particules par le courant ascendant d'un fluide (l'air à température négative de par exemple -25°C) dont la vitesse va équilibrer le poids du produit solide et va assurer une expansion de la couche de particules solides individualisées et mobiles en un lit fluidisé.

On note, lors de la surgélation en lit fluidisé, une augmentation de matières sèches des particules de levure comprise entre 0 et 1 point de matières sèches.

Les vermicelles de levures sont surgelés entre -18°C et -20°C en environ 20 à 30 minutes et emballés d'une part sous air, d'autre part sous gaz neutre après surgélation.

Ces sacs sont conservés à -20°C, on mesure la force de la levure fraîche de départ et de la levure surgelée à environ 77% de matières sèches obtenues après un mois, deux mois et trois mois de conservation à -20°C.

Les activités fermentatives obtenues, mesurées au fermentomètre de Burrows et Harrison selon le test A₁ décrit dans les brevets U.S. No 4.370.420 ou 4.396.632, restent pour les levures sèches surgelées à 77% de matières sèches en conservation dans une fourchette comprise entre 97% et 101% de la valeur obtenue pour la même quantité de matières sèches de la levure fraîche à 33% de matières sèches qui a été utilisée pour obtenir les susmentionnées levures surgelées. On ne note aucune déperdition significative d'activité fermentative.

La levure surgelée à 77% de matières sèches en vermicelles reste fluide, c'est-à-dire que les particules des vermicelles sont très aisées à désagréger et donc à manutentionner et à utiliser. Elles ont une bonne fluidité.

### EXEMPLE 2

On prépare, comme dans l'exemple 1, une levure surgelée en vermicelles de diamètre 0,5 à 1 mm, à environ 77% de matières sèches. On part d'une levure pressée à environ 7% d'azote sur matières sèches et à plus de 15% de tréhalose sur matières sèches. On conserve la levure surgelée obtenue pendant 11 semaines à -20°C.

Dans le cadre de la préparation de la levure surgelée ci-dessus, on prélève un échantillon de la levure à environ 77% de matières sèches avant surgélation et on le conserve pendant 11 semaines à +4°C.

On prend une levure américaine du commerce en pain d'une livre, prélevée très près de sa sortie usine et on la transporte par avion sur le lieu de l'essai à +4°C. Cette levure pressée avait, au moment de l'essai de fabrication de pâtes congelées décrit ci-dessous, une matière sèche de 30% et une teneur en azote sur matières sèches de 9,1%.

On prend une levure sèche instantanée du commerce de bonne qualité à 95% de matières sèches.

Ces quatre levures sont testées dans un essai de panification type U.S. White-Bread, schéma No-time dough à partir de pâtes congelées. Les quatre levures sont incorporées directement en mélange avec la farine, ce qui est très pénalisant pour la levure sèche instantanée qui va se trouver en contact avec une eau de coulage à 3°C et une pâte très froide. Une levure sèche instantanée réhydratée dans de bonnes conditions à au moins 30°C et ayant pu reconstituer ses membranes cellulaires ferait au moins jeu égal avec la levure fraîche témoin. La levure surgelée à environ 77% de matières sèches, conservée 11 semaines à -20°C est incorporée directement sans décongélation préalable.

La formule U.S. White-Bread retenue a la composition suivante :
farine U.S. 100 parties en poids
eau 57 parties en poids
sel 2,25 parties en poids
matières sèches levure 1,6 parties parties en poids
sucre 8 parties en poids
shortening 5 parties en poids
Panodan 90 0,3 parties en poids
(ester diacétyl tartrique de monoglycérides fabriqué par GRINDSTED)
acide ascorbique 100 ppm
bromate 50/50 20 ppm (bromate pur: 10 ppm)

La température des pâtes fin pétrissage est de 15°C et les pâtes obtenues sont immédiatement placés dans une enceinte à -40°C après façonnage de manière à les congeler à -20°C à coeur. La durée entre la fin du pétrissage et la fin du façonnage et donc le début de la congélation est maintenue constante et égale à 25 minutes. Les pâtons congelés obtenus sont conservés à -20°C. On décongèle les pâtons au bout de une, quatre, huit, douze et seize semaines dans une enceinte à +26°C où ils sont portés à 4°C à coeur en 135 minutes. Ensuite, on mesure la durée de proof-time pour un développement de pâte à volume constant, en étuve à 43°C et 94% d'humidité relative.

On obtient les résultats suivants :

| Proof-time exprimé en minutes | | | | |
|---|---|---|---|---|
| | Levure fraîche US témoin | Levure sèche témoin | Levure à 77% de matières sèches, conservée 11 semaines à 4°C | Levure à 77% de matières sèches, surgelée conservée 11 semaines à -20°C |
| 1 semaine | 105 | 145 | 110 | 95 |
| 4 semaines | 115 | 158 | 125 | 100 |
| 8 semaines | 124 | 168 | 131 | 102 |
| 12 semaines | 136 | 175 | 138 | 110 |
| 16 semaines | 145 | 182 | 150 | 118 |

On note en moyenne sur plusieurs essais une augmentation du proof-time exprimé en minutes par semaine de conservation à -20°C des pâtes congelées :
levure fraîche US témoin + 2,6 minutes/semaine
levure sèche témoin + 2,4 minutes/semaine
levure surgelée, finement divisée et fluide à 77% de matières sèches, conservée 11 semaines à -20°C + 1,6 minute/semaine.

### EXEMPLE 3

On prépare, comme dans l'exemple 1, une levure surgelée ou congelée en vermicelles de diamètre 0,5 à 1 mm, à environ 77% de matières sèches. La souche utilisée est la souche NCYC Nₒ995 décrite dans le brevet US Nₒ 4.396.632.

La culture de cette souche est conduite essentiellement de manière à obtenir une teneur relativement élevée en azote, égale ou supérieure à 8%. L'émulsifiant ajouté à la levure pressée est le monostéarate de sorbitan à une dose de 0,3% par rapport aux matières sèches levures mises en oeuvre.

L'analyse de la levure surgelée en particules fluides obtenue donne (résultats de 8 essais):
Matières sèches 74 à 80% moyenne 77%
Azote sur matières sèches 7,9 à 8,6% moyenne 8,2%
Force fermentative mesurée en 2 heures au fermentomètre de Burrows et Harrison selon le susdit test A₁ décrit notammentmoyenne dans le brevet US 4.396.632 159 à 170 ml 162 ml de CO₂

La levure surgelée en particules fluides obtenue a une activité fermentative du même ordre de grandeur que celle des levures fraîches compressées, très actives et une conservation au moins égale à celle des meilleures levures sèches, et une activité supérieure de l'ordre de 25% à celle de ces levures sèches.

### EXEMPLE 4

On prépare, comme dans l'exemple 1, une levure surgelée ou congelée en vermicelles de diamètre 0,5 à 1 mm, à environ 77% de matières sèches.

La souche utilisée est une souche osmotolérante du groupe des souches NCYC R 30, NCYC 878, NCYC 996 ou NCYC 890 décrites dans les brevets US No 4.328.250, 4.318.930, 4.396.632.

Ces souches sont multipliées comme indiqué dans lesdits brevets U.S. L'émulsifiant utilisé est le monostéarate de sorbitan à une dose de 0,3%.

La levure surgelée en particules fluides obtenue à environ 77% de matières sèches a une activité au moins égale à 30 ml dans le test A₄ sur 1 heure et, de préférence, au moins égale à 40 ml de CO₂ dans le test A₄ sur 1 heure décrit dans les susdits brevets U.S.

Cette levure surgelée en particules fluides est utilisée pour la fabrication de pâtisseries danoises de formule type:
Farine 100 parties en poids
Sucre 15 parties parties en poids
Matières grasses 15 parties parties en poids
Levure pressée US 15 parties en poids + sels, oxydants et émulsifiants
Eau 50 parties en poids.

Le pétrissage est effectué en trois étapes dans un pétrin horizontal à double enveloppe contenant de l'eau glacée à -18°C. La température de la pâte au départ est de 0°C et à la fin du pétrissage 10°C maximum.

Dans un tel procédé, il est très difficile d'employer des levures sèches à moins de réhydrater cette levure à au moins 20°C et après 1 heure, refroidir la crème de levure obtenue.

La levure surgelée en particules fluides peut être incorporée directement dans la pâte à une dose de 3 à 5%, en remplacement des 15% de levure fraîche pressée pour obtenir au moins le même développement de la pâte.

## Revendications

1. Levure de panification surgelée ou congelée en particules fluides s'écoulant librement, ayant une matière sèche entre 70 et 85%, conservant pratiquement indéfiniment et au moins pendant trois mois les propriétés de la levure fraîche de départ et susceptible d'être obtenue par:
- division d'une levure fraîche, riche en tréhalose et ayant ses membranes intactes, par extrusion en particules individuelles de moins de 3 mm et, de préférence, de moins de 1 mm,
- séchage des particules ainsi obtenues jusqu'à une teneur en matières sèches comprise entre 70 et 85% par un séchage ménageant,
- refroidissement rapide des particules séchées jusqu'à une température comprise entre -1° et +4°C,
- surgélation ou congélation des particules ainsi refroidies par fluidisation dans un courant d'air à température négative comprise entre -10° et -40°C.

2. Levure surgelée ou congelée de panification selon la revendication 1, ayant une matière sèche comprise entre 72 et 80%.

3. Levure de panification selon l'une des revendications 1 et 2, caractérisée par le fait qu'elle comporte un ou plusieurs additifs choisis parmi les additifs de séchage, les antimottants ou les agents cryoprotecteurs.

4. Levure de panification selon l'une des revendications 1 à 3, caractérisée par le fait que, dans les tests A₁, elle donne un dégagement gazeux compris entre 159 et 170 ml en deux heures.

5. Procédé de fabrication de levures surgelées ou congelées se présentant sous forme de particules fluides s'écoulant librement, caractérisé par le fait:
- qu'on divise une levure fraîche riche en tréhalose et ayant ses membranes intactes, par extrusion en particules individuelles de moins de 3 mm, de préférence de moins de 1 mm,
- qu'on sèche les particules ainsi obtenues jusqu'à une teneur en matières sèches comprise entre 70 et 85% par un séchage ménageant,
- qu'on refroidit rapidement les particules séchées à une température comprise entre -1° et +4°C,
- qu'on surgèle ou congèle les particules refroidies par fluidisation dans un courant d'air à une température négative comprise entre -10° et -40°C.

6. Procédé selon la revendication 5, caractérisé par le fait que le séchage des particules de levures est poursuivi jusqu'à une teneur en matières sèches comprise entre 72 et 80%.

7. Procédé selon l'une des revendications 5 et 6, caractérisé par le fait qu'avant l'extrusion on ajoute à la levure un ou plusieurs additifs choisis parmi les additifs de séchage, les agents antimottants et les agents cryoprotecteurs.

8. Procédé de fabrication de pâtes congelées caractérisé par le fait que l'on a recours à une levure conforme à l'une des revendications 1 à 4.

## Patentansprüche

1. Tiefgekühlte oder gefrorene Bäckerhefe in Form von freifließenden Teilchen mit einer Trockensubstanz zwischen 70% und 85%, wobei diese Bäckerhefe die Eigenschaften der der anfänglichen Frischhefe auf praktisch unbestimmte Zeit und zumindest während drei Monaten beibehält und erhältlich ist durch:
- Zerteilung der Frischhefe, die trehalosereich ist und deren Membranen intakt sind, durch Extrudieren in Einzelteilchen von weniger als 3 mm und vorzugsweise von weniger als 1 mm,
- Trocknung der so erhaltenen Teilchen bis auf einen Trockensubstanzgehalt zwischen 70 und 85% durch schonendes Trocknen,
- schnelle Abkühlung der getrockneten Teilchen bis auf eine Temperatur zwischen -1°C und +4°C,
- Tiefkühlung oder Gefrieren der so abgekühlten Teilchen durch Fluidisierung in einem Luftstrom bei einer negativen Temperatur zwischen -10° und -40°C.

2. Bäckerhefe nach Anspruch 1, dadurch gekennzeichnet, daß sie eine Trockensubstanz zwischen 72 und 80% besitzt.

3. Bäckerhefe nach einem der Ansprüche 1 und 2, dadurch gekennzeichnet, daß sie einen oder mehrere Zusatzstoffe enthält ausgewählt aus Trocknungsmitteln, antiklumpenbildenden Mitteln oder Cryoschutzmitteln.

4. Bäckerhefe nach einem der Ansprüche 1 bis 3, dadurch gekennzeichnet, daß sie in den Testen A1 zu einer Gasfreisetzung zwischen 159 und 170 ml in zwei Stunden führt.

5. Verfahren zur Herstellung tiefgekühlter oder gefrorener Hefen in Form freifließender Teilchen, dadurch gekennzeichnet, daß man
- eine trehalosereiche Frischhefe mit intakten Membranen durch Extrudieren in Einzelteilchen von weniger als 3 mm, vorzugsweise von weniger als 1 mm zerteilt,
- die so erhaltenen Teilchen bis auf einen Trockensubstanzgehalt zwischen 70 und 85% durch schonendes Trocknen trocknet,
- die getrockneten Teilchen schnell auf eine Temperatur zwischen -1°C und +4°C abkühlt,
- die abgekühlten Teilchen durch Fluidisierung in einem Luftstrom bei einer negativen Temperatur zwischen -10° und -40°C tiefkühlt oder gefriert.

6. Verfahren nach Anspruch 5, dadurch gekennzeichnet, daß die Trocknung der Hefeteilchen bis auf einen Trockensubstanzgehalt zwischen 72 und 80% erfolgt.

7. Verfahren nach einem der Ansprüche 5 und 6, dadurch gekennzeichnet, daß man vor dem Extrudieren der Hefe einen oder mehrere Zusatzstoffe zufügt, ausgewählt unter den Trocknungsmitteln, den antiklumpenbildenden Mitteln und den Cryoschutzmitteln.

8. Verfahren zur Herstellung von tiefgefrorenen Teigen, dadurch gekennzeichnet, daß man eine Hefe nach einem der Ansprüche 1 bis 4 verwendet.

## Claims

1. Frozen baker's yeast in the form of free-flowing particles having a dry matter content comprised between 70% and 85%, preserving practically indefinitely and at least during three months the properties of the initial fresh yeast and obtainable by:
- dividing by extrusion a fresh yeast, rich in trehalose and having its membranes intact, into individual particles of less than 3 mm and, prefereably, of less than 1 mm,
- drying the thus obtained particles until a dry matter content comprised between 70 and 85% by gentle drying,
- quickly cooling of the dried particles until a temperature comprised between -1° and +4°C,
- freezing of the particles thus cooled by fluidization in an air flow at a negative temperature comprised between -10° and -40°C.

2. Frozen baker's yeast according to claim 1, having a dry matter content comprised between 72% and 89%.

3. Baker's yeast according to one of claims 1 and 2, characterized by the fact that it comprises one or several additives selected from the drying agents, the anticlumping agents or the cryoprotective agents.

4. Baker's yeast according to one of claims 1 to 3, characterized by the fact that, in tests A₁, it gives a gas release comprised between 159 and 170 ml in two hours.

5. Process for the manufacture of frozen baker's yeast in the form of free-flowing particles, characterized by the fact:
- that a fresh yeast, rich in trehalose and having its membranes intact, is divided by extrusion into individual particles of less than 3 mm and, prefereably, of less than 1 mm,
- that the thus obtained particles are dried until a dry matter content comprised between 70 and 85% by gentle drying,
- that the dried particles are quickly cooled to a temperature comprised between -1° and +4°C,
- that the particles thus cooled are frozen by fluidization in an air flow at a negative temperature comprised between -10° and -40°C.

6. Process according to claim 5, characterized by the fact that the drying of the yeasts particles is continued until a dry matter content comprised between 72 and 80% is obtained.

7. Process according to one of claims 5 and 6, characterized by the fact that there is added to the yeast before its extrusion one or several additives selected from the drying agents, the anticlumping agents and the cryoprotective agents.

8. Process for the manufacture of frozen doughs characterized by the fact that there is used a yeast according to one of claims 1 to 4.
